# EUROPÄISCHE PATENTSCHRIFT

(11) **EP 1 862 131 B1**
(45) Veröffentlichungstag und Bekanntmachung des Hinweises auf die Patenterteilung: **08.07.2009**
(21) Anmeldenummer: 07107081.7
(22) Anmeldetag: 27.04.2007
(51) Int. Cl.: A61B 17/132

(54) **Venenstauvorrichtung**
Vein occluding device
Dispositif d'occlusion veineuse

(30) Priorität: 31.05.2006 DE 102006025786
(43) Veröffentlichungstag der Anmeldung: 05.12.2007
(73) Patentinhaber: Prämeta GmbH & Co. KG, 53842 Troisdorf (DE)
(72) Erfinder: Heisig, Rolf, 72768, Reutlingen (DE); Pyschik, Roman, 42859, Remscheid (DE)
(74) Vertreter: Dallmeyer, Georg

(56) Entgegenhaltungen:
- EP-A- 0 513 485
- EP-A1- 0 374 464
- DE-A1- 3 431 728

## Beschreibung

Die Erfindung bezieht sich auf eine Venenstauvorrichtung für Körperteile mit einem Schlossgehäuse und mit einem in dem Schlossgehäuse um eine Schwenkachse zwischen Seitenwangen des Schlossgehäuses schwenkbar gelagerten Klemmhebel für ein durch das Schlossgehäuse hindurchgeführte Band, wobei das andere Bandende mittels eines Schuhes o.dgl. am Klemmhebel einrastbar oder durch das Schlossgehäuse ebenfalls hindurchführbar ist.

Die bekannten Venenstauvorrichtungen bzw. Abschnürvorrichtungen für Körperteile sind üblicherweise so ausgebildet, dass zur schwenkbaren Lagerung des Klemmhebels ein Achsstift vorgesehen ist, der beiderseits in den Seitenwangen des Schlossgehäuses gelagert ist. Hierbei weisen die Seitenwangen Bohrungen auf, die bis zur Aussenfläche der Seitenwangen führen, so dass der Achsstift von aussen quer durch das Schlossgehäuse hindurchgeführt werden kann. Die äusseren Flächen der Seitenwangen des Schlossgehäuses wurden in der Weise bearbeitet, dass die Stirnflächen des Achsstiftes mit den Aussenflächen der Seitenwangen bündig verlaufen. Der Achsstift ist von aussen zwar sichtbar, aber die Aussenflächen der Seitenwangen bleiben glatt.Es hat sich herausgestellt, dass bei vielem Gebrauch der Abschnürvorrichtung der Achsstift nicht immer in seiner Lage verbleibt, sondern sich nach der einen oder der anderen Richtung verschiebt, so dass er an den Aussenflächen der Seitenwangen entweder vorsteht oder zurücksteht. Beides ist bei der Handhabung im ärztlichen Bereich unbefriedigend. In dem einen Fall bietet der vorstehende Achsstift die Möglichkeit einer leichten Verletzung der Haut der Bedienungsperson. Das Zurückstehen des Achsstiftes führt zu einer Ausnehmung, in der sich Bakterien o.dgl. festsetzen können.

Die EP 0 513 485 A beschreibt eine Vorrichtung zum Abschnüren von Gliedmaßen, einen sogenannten Venenstauer, die ein Abschnürband aufweist, welches mit seinem einen Ende mittels eines Steckers lösbar in einem Bandschloss befestigbar ist, und dessen anderes Ende unter Schlaufenbildung durch das Bandschloss zurückgeführt ist und in diesem mittels einer Klemmwippe arretierbar ist, die bei Ausübung einer Zugkraft auf den Stecker über ein Zwischenteil automatisch betätigbar ist, welches die Klemmwippe im hinteren Abschnitt untergreift. Zum Aufheben der Klemmwirkung ist als separates Bauteil eine Drucktaste vorgesehen, die schwenkbar unter einem Quersteg im Bandschloss abgestützt ist und mit einem Druckpunkt auf dem hinteren Teil der Klemmwippe aufliegt. Mittels einer Schiebetaste lässt sich die Steckverbindung lösen.

Die DE 34 31 728 A offenbart eine Schnalle eines Gefäßstauers bestehend aus einem, das eine Gurtende in einer Führung längsverschiebbar aufnehmenden Unterteil und aus einem das andere Gurtende fest tragenden und in eine Querachse des Unterteils einrastbaren, selbständigen Oberteil. Das unter Federwirkung stehende Oberteil dient als Klemmlasche für das in dem Unterteil längsverschiebbare Gurtende. Das Unterteil weist als U-förmig offenes Gehäuse einen Einsatz auf, der alleiniger Träger für die Querachsen, die Federn und die Mittel zum Befestigen des Einsatzes an dem Unterteil ist.

Die DE 38 39 794 und EP-0 374 464-A1 beschreiben einen Venenstauer ohne einen sichtbaren Achsstift. Die Achse für den in dem Schlossgehäuse fest integrierten Klemmhebel greift in Nuten in den inneren Gehäuse-Seitenwangen ein und wird mit Hilfe von Füllelementen, z.B. Keile, verschlossen.

Wie schon im Falle einer sichtbaren Achse sind auch die Füllelemente nach der DE 38 39 794 von außen sichtbar. Sie können ebenso vor- oder zurückstehen und erschweren so die Handhabung des Venenstauers. Aus der Sicht der Hygiene können sich an den Kanten und in den Ausnehmungen Bakterien festsetzen.

Der Erfindung liegt die Aufgabe zugrunde, bei einem Venenstauer mit einem fest integrierten Klemmhebel die Verletzungsgefahr zu verringern und die hygienischen Bedingungen weiter zu verbessern.

Zur Lösung dieser Aufgabe dienen die Merkmale des Anspruchs 1.

Die Erfindung sieht in vorteilhafter Weise vor, dass Achsstutzen für den in das Schlossgehäuse fest integrierten Klemmhebel in in das Gehäuse nachträglich eingeprägte Ausnehmungen, vorteilhafterweise runde Sacklöcher eingreifen, und dass keine sichtbare Achse und keinerlei Hilfs- oder Füllelemente sichtbar sind. Alle Außenflächen sind geschlossene Flächen, die keinen Grund zur Beeinträchtigung bei der Handhabung geben. Aus hygienischer Sicht können sie leicht sauber gehalten werden.

Die Achse oder die Achsstutzen zur Lagerung des Klemmhebels sind an den Seiten vorstehend und für den Zweck der Montage in das Schlossgehäuse gefedert oder verschiebbar ausgeführt.

Vorzugsweise sind die in dem Klemmhebel axial beweglich gelagerten Achsstutzen durch eine Feder, insbesondere durch eine Spiralfeder oder eine gekrümmte Blattfeder nach außen axial vorgespannt. Aufgrund der Federkraft können die Achsstutzen in die Ausnehmungen einrasten, so dass der Klemmhebel sicher in dem Schlossgehäuse gehalten ist.

Der Klemmhebel kann eine einteilige, axial längenveränderliche Achse aufweisen, deren gegenüber dem Klemmhebel überstehende Enden in dem Klemmhebel axial beweglich gelagerte, nach innen eindrückbare Achsstutzen bilden. Eine derartige Ausführungsform hat den Vorteil, die Anzahl der Teile zu reduzieren und einfach montierbar zu sein.

Die einteilige Achse des Klemmhebels kann aus einer omega-förmigen Feder, einer O-förmigen Feder oder einer Spiralfeder bestehen, die eine axiale Federkraft erzeugt, die die aus dem Klemmhebel seitlich vorstehenden und als Achsstutzen dienenden freien Enden der Feder nach außen vorspannt.

Bei einer alternativen Ausführungsform ist vorgesehen, dass die Achsstutzen des Klemmhebels einstückig mit dem Klemmhebel sind und axial federnd an den den Seitenwangen des Schlossgehäuses gegenüberliegenden Seiten des Klemmhebels ausgebildet sind. Der Vorteil dieser Ausführungsform besteht darin, dass der Montageaufwand reduziert ist.

Die Achsstutzen des Klemmhebels können dabei einstückig an dem Klemmhebel angeformten federnden Schenkel angeordnet sein.

Ausführungsbeispiele der Erfindung werden anhand der Zeichnungen näher erläutert.

Es zeigen:
- Fig. 1: eine schematische Darstellung einer Ausführungsform einer Venen-stauvorrichtung gemäß der Erfindung im Längsschnitt,
- Fig. 2: eine Stirnansicht auf das Schlossgehäuse,
- Fig. 3a bis Fig. 3c: zwei Verfahren zum Einprägen der Achsaufnahmen,
- Fig. 4 bis Fig. 9: unterschiedliche Ausführungsformen der federnden Achsstutzen in einer Draufsicht.

Die Venenstauvorrichtung 1 bzw. die Abschnürvorrichtung für Körperteile weist ein Schlossgehäuse 2 auf, in dem ein Klemmhebel 3 zwischen Seitenwangen 2a, 2b um eine Schwenkachse 4 schwenkbar gelagert ist. In dem Klemmhebel 3 kann ein Halteorgan 5 schnappend eingerastet werden, an dem das Bandende 6 des Bandes 7 befestigt ist, wobei die Einraststellung des Halteorganes 5 durch eine Feder 8 unterstützt wird. Das andere Bandende 9 ist durch das Schlossgehäuse 2 hindurchgeführt.

Das Schlossgehäuse 2 weist in die Seitenwangen 2a, 2b auf deren Innenseite eingeprägte Ausnehmungen 13, 14 als Achsaufnahmen auf. In diese Achsaufnahmen können in der Schwenkachse 4 verlaufende axial beweglich in dem Klemmhebel 3 gelagerte Achsstutzen 11, 12 unter Federkraft einrasten. Die eingeprägten Ausnehmungen 13, 14 sind so eingeprägt, dass sie vor den Außenflächen der Seitenwangen 2a, 2b des Schlossgehäuses 2 enden, so dass die Außenflächen der Seitenwangen massiv geschlossen ausgebildet sind und aufgrund der glatten Außenfläche keine Ansatzstellen zum Einnisten von Bakterien bilden.

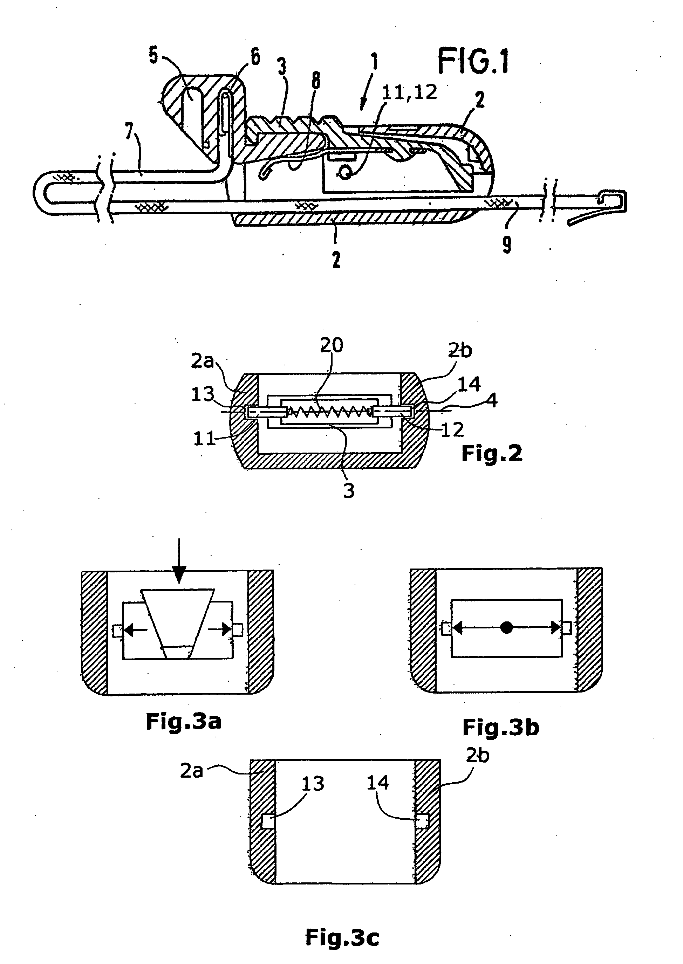

Fig. 2 zeigt ein bevorzugtes Ausführungsbeispiel, bei denen die Achsstutzen 11, 12 mit Hilfe einer Spiralfeder 20 axial nach außen vorgespannt werden, so dass sie in die eingeprägten Ausnehmungen 13, 14 einrasten können. Wie aus Fig. 2 ersichtlich, sind die Achsstutzen 11, 12 in der Schwenkachse 14 axial beweglich in dem Klemmhebel 3 gelagert. Es versteht sich, dass die Achsstutzen 11, 12 an ihren innenseitigen Enden einen Bund aufweisen können, damit sie aus dem Klemmhebel 3 nicht herausfallen können. Zum Aufnehmen der Spiralfeder 20 können die inneren Enden der Achsstutzen 11, 12 beispielsweise hülsenförmig ausgebildet sein.

Die Fign. 3a und 3b zeigen unterschiedliche Vorgehensweisen zum Prägen der Ausnehmungen 13, 14 auf den Innenseiten der Seitenwangen 2a, 2b.

Fig. 3a zeigt ein Werkzeug für eine parallele und gleichzeitige Prägung beider Ausnehmungen 13, 14 während Fig. 3b ein Werkzeug für eine serielle Prägung zeigt.

Die Ausnehmungen 13, 14 in den inneren Flächen der Seitenwangen 2a, 2b des Schlossgehäuses 2 werden mit Hilfe derartiger Prägewerkzeuge erzeugt. Fig. 3c zeigt die Ausnehmungen 13, 14 nach dem Prägevorgang.

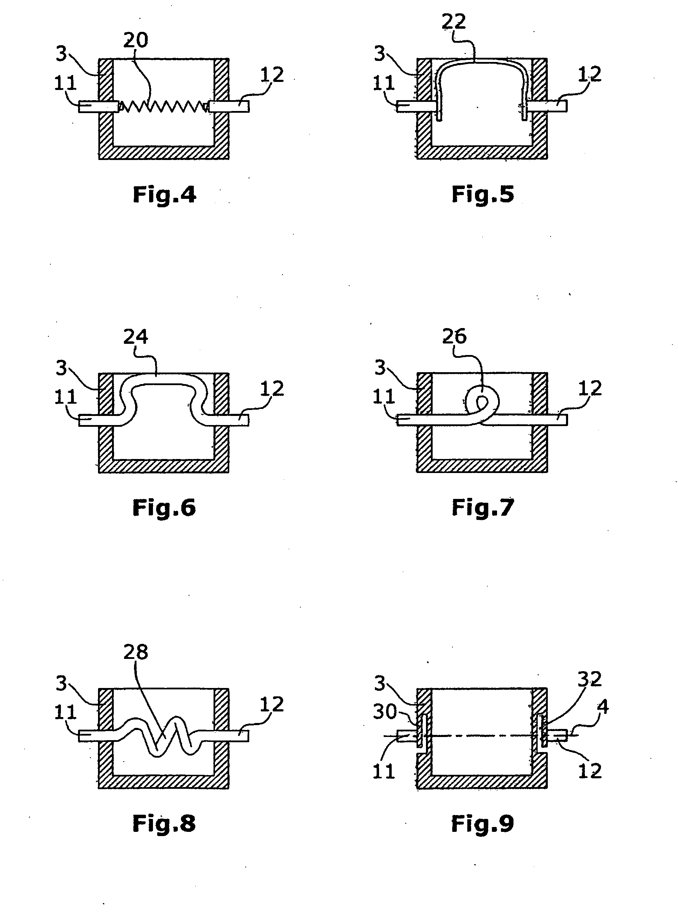

Fig. 4 zeigt eine einteilige Achse, deren Achsstutzen 11, 12 von einer Spiralfeder 20 nach außen durch eine Lagerbohrung des Klemmhebels 3 gedrückt werden.

Fig. 5 zeigt eine Ausführungsform mit einer gekrümmten Blattfeder 22, die die Achsstutzen 13, 14 axial nach außen vorspannt.

Die Fign. 6, 7 und 8 zeigen eine einteilige Achse, die durch eine Feder gebildet ist, wobei Fig. 6 eine omega-förmige Feder 24 zeigt, Fig. 6 eine O-förmige Feder 26 und Fig. 7 eine Spiralfeder 28.

Die freien Enden dieser Federn 24, 26, 28 bilden die Achsstutzen 11, 12 einer axial längenveränderlichen einteiligen Achse.

Fig. 9 zeigt ein Ausführungsbeispiel, bei dem die Achsstutzen 11, 12 einstückig mit dem Klemmhebel 3 sind. Dabei sind die Achsstutzen 11, 12 an federnden Schenkeln 30, 32 angeformt, die ein Einfedern der Achsstutzen 11, 12 in der Schwenkachse 4 nach innen ermöglichen, so dass die Achsstutzen 11, 12 beim Einsetzen in das Schlossgehäuse 2 zunächst einfedern können und dann in die geprägten Ausnehmungen 13, 14 einrasten können.

## Patentansprüche

1. Venenstauvorrichtung (1) für Körperteile, mit einem Schlossgehäuse (2) und mit einem in dem Schlossgehäuse (2) um eine Schwenkachse (4) zwischen Seitenwangen (2a, 2b) des Schlossgehäuses (2) schwenkbar gelagerten Klemmhebel (3) für ein durch das Schlossgehäuse hindurchgeführtes Band (7), wobei das andere Bandende (6) mittels eines Halteorganes (5) am Klemmhebel (3) einrastbar oder durch das Schlossgehäuse (2) ebenfalls hindurchführbar ist, wobei das Schlossgehäuse (2) in die Seitenwangen (2a, 2b) auf der Innenseite eingeprägte Ausnehmungen (13, 14) als Achsaufnahmen aufweist, und wobei der Klemmhebel (3) in der Schwenkachse (4) verlaufende Achsstutzen (11,12) aufweist,
**dadurch gekennzeichnet,**
**dass** die in der Schwenkachse (4) verlaufenden Achsstutzen (11, 12) axial beweglich gelagert sind und unter Federkraft in die Ausnehmungen (13, 14) einrastbar sind.

2. Venenstauvorrichtung nach Anspruch 1, **dadurch gekennzeichnet, dass** die in dem Klemmhebel (3) axial beweglich gelagerten Achsstutzen (11, 12) durch eine Feder, insbesondere durch eine Spiralfeder (20) oder eine gekrümmte Blattfeder (22), nach außen axial vorgespannt sind.

3. Venenstauvorrichtung nach Anspruch 1, **dadurch gekennzeichnet, dass** der Klemmhebel (3) eine einteilige axial längenveränderliche Achse aufweist, deren gegenüber dem Klemmhebel (3) überstehende Enden in dem Klemmhebel (3) axial beweglich gelagerte, nach innen eindrückbare Achsstutzen (11, 12) bilden.

4. Venenstauvorrichtung nach Anspruch 3, **dadurch gekennzeichnet, dass** die einteilige Achse des Klemmhebels (3) aus einer omega-förmigen Feder (24), einer O-förmigen Feder (26) oder einer Spiralfeder (28) besteht, die eine axiale Federkraft erzeugt, die die aus dem Klemmhebel (3) seitlich vorstehenden und als Achsstutzen (11, 12) dienenden freien Enden der Feder (24, 26, 28) nach außen vorspannt.

5. Venenstauvorrichtung nach Anspruch 1, **dadurch gekennzeichnet, dass** die Achsstutzen (11, 12) des Klemmhebels (3) einstückig mit dem Klemmhebel (3) sind und axial federnd an den den Seitenwange (2a, 2b) des Schlossgehäuses (2) gegenüberliegenden Seiten des Klemmhebels (3) ausgebildet sind.

6. Venenstauvorrichtung nach Anspruch 5, **dadurch gekennzeichnet, dass** die Achsstutzen (11, 12) des Klemmhebels (3) an einstückig an dem Klemmhebel angeformten federnden Schenkeln (30, 32) angeordnet sind.

## Claims

1. A tourniquet device (1) for body parts, comprising a lock housing (2) and comprising a clamping lever (3) for a strap (7) passed through said lock housing, said clamping lever being supported for pivoting around a pivot axis (4) between side cheeks (2a, 2b) of the lock housing (2), wherein the other end (6) of the strap is either adapted to engage the locking lever (3) by means of a retaining member (5) or may also be passed through the lock housing (2), said lock housing (2) comprising recesses (13, 14) embossed on the inner surface of the side cheeks (2a, 2b) as axis seats, and wherein said clamping lever (3) has axle stubs (11, 12) extending on the pivot axis (4),
**characterized in that**
the axle stubs (11, 12) extending on the pivot axis (4) are supported for axial movement and are adapted to engage into the recesses (13, 14) under spring force.

2. The tourniquet device of claim 1, **characterized in that** the axle stubs (11, 12) supported for axial movement in the clamping lever (3) are biased axially outward by means of a spring, especially a spiral spring (20) or a bent leaf spring (22).

3. The tourniquet device of claim 1, **characterized in that** the clamping lever (3) has an integral axis axially variable in length, whose ends protruding beyond the clamping lever (3) form axle stubs (11, 12) that are supported for axial movement and adapted to be pushed inward.

4. The tourniquet device of claim 3, **characterized in that** the integral axis of the clamping lever (3) consists of an omega-shaped spring (24), an O-shaped spring (26) or a spiral spring (28), generating an axial spring force that biases outward the free ends of the spring (24, 26, 28) laterally protruding from the clamping lever (3) and serving as axle stubs (11, 12).

5. The tourniquet device of claim 1, **characterized in that** the axle stubs (11, 12) of the clamping lever (3) are formed integrally with the clamping lever (3) and are formed in an axially resilient manner at the sides of the locking lever (3) opposite the side cheeks (2a, 2b) of the lock housing (2).

6. The tourniquet device of claim 5, **characterized in that** the axle stubs (11, 12) of the clamping lever (3) are arranged on resilient legs (30, 32) formed integrally with the clamping lever.

## Revendications

1. Tourniquet (1) pour des parties du corps, comprenant un boîtier de serrure (2) et un levier de serrage (3) pour une bande (7) passée à travers ledit boîtier de serrure, ledit levier étant supporté de manière pivotable autour d'un axe de pivotement (4), dans le boîtier de serrure (2) entre des mâchoires latérales (2a, 2b) dudit boîtier de serrure (2), l'autre extrémité (6) de la bande peut être encliquetée audit levier de serrage (3) par un moyen de retenue (5) ou peut aussi être passée à travers le boîtier de serrure (2), le boîtier de serrure (2) comprenant des creux (13, 14) estampés dans la face interne des mâchoires latérales (2a, 2b) comme logements de l'axe, et le levier de serrage (3) comprenant des tourillons (11, 12) s'étendant dans l'axe de pivotement (4),
**caractérisé en ce que**
les tourillons (11, 12) s'étendant dans l'axe de pivotement (4) sont supportés pour mouvement axial et sont aptes à être encliquetés dans les creux (13, 14) par effort de ressort.

2. Tourniquet selon la revendication 1, **caractérisé en ce que** les tourillons (11, 12) supportés de manière mobile selon la direction axiale dans ledit levier de serrage (3) sont précontraints axialement vers l'extérieur par un ressort, en particulier un ressort spiral (20) ou un ressort à lames (22) courbé.

3. Tourniquet selon la revendication 1, **caractérisé en ce que** le levier de serrage (3) comprend un axe unitaire, axialement variable en longueur, dont les extrémités s'étendant au-delà du levier de serrage (3) formant des tourillons (11, 12) supporté dans ledit levier de serrage (3) de manière mobile dans la direction axiale et aptes à être poussés vers l'intérieur.

4. Tourniquet selon la revendication 3, **caractérisé en ce que** l'axe unitaire dudit levier de serrage (3) est formé par un ressort (24) de forme oméga, un ressort (26) en forme O, ou un ressort spiral (28), générant un effort de ressort axial qui précontraint vers l'extérieur les extrémités libres du ressort (24, 26, 28) saillant latéralement dudit levier de serrage (3) et servant comme tourillons (11, 12).

5. Tourniquet selon la revendication 1, **caractérisé en ce que** les tourillons (11, 12) dudit levier de serrage (3) sont d'un seul tenant avec ledit levier de serrage (3) et sont formés axialement résilient aux faces du levier de serrage (3) opposées aux mâchoires latérales (2a, 2b) dudit boîtier de serrure (2).

6. Tourniquet selon la revendication 5, **caractérisé en ce que** les tourillons (11, 12) du levier de serrage (3) sont prévus à des ailes (30, 32) résilientes formées d'un seul tenant avec le levier de serrage.
